# EUROPÄISCHE PATENTSCHRIFT

(11) **EP 1 536 736 B1**
(45) Veröffentlichungstag und Bekanntmachung des Hinweises auf die Patenterteilung: **14.03.2007**
(21) Anmeldenummer: 00954630.0
(22) Anmeldetag: 16.08.2000
(51) Int. Cl.: A61B 18/14, A61N 1/06

(54) **Verfahren zur Herstellung eines Katheters mit verbesserten elektrischen Eigenschaften**
Method of producing a catheter with enhanced electrical characteristics
Procédé de fabrication d'un cathéter avec des qualités electriques améliorées

(30) Priorität: 17.08.1999 DE 19944805
(43) Veröffentlichungstag der Anmeldung: 08.06.2005
(73) Patentinhaber: Muntermann, Axel, 35578 Wetzlar (DE)
(72) Erfinder: Muntermann, Axel, 35578 Wetzlar (DE)
(74) Vertreter: Herden, Andreas F.
(86) Internationale Anmeldenummer: PCT/EP2000/007942
(87) Internationale Veröffentlichungsnummer: WO 2001/012091

(56) Entgegenhaltungen:
- DE-A- 19 628 879
- DE-A- 19 740 976
- FR-A- 2 653 655
- US-A- 4 653 500
- US-A- 5 810 764

## Beschreibung

Die Erfindung betrifft ein Verfahren zur Herstellung eines Katheters mit verbesserten elektrischen Eigenschaften.
Bei der Katheterablation myokardialen Gewebes ist es eines der Hauptziele, durch Läsionen der oberen Schichten des Herzgewebes Reizleitungsbereiche zu unterbrechen, welche die Herztätigkeit negativ beeinflussen können. Der Erfolg einer Behandlung hängt jedoch ganz wesentlich davon ab, ob bei der Ablation die richtige Läsionstiefe erreicht wurde. Richtige Läsionstiefe bedeutet hierbei im wesentlichen, daß die unerwünschten, die Reizleitung störenden Bereiche ausgeräumt, aber keine darüber hinaus gehenden Schädigungen eingebracht wurden. Es ist offensichtlich, daß bei zu geringer Läsionstiefe der Behandlungserfolg gefährdet ist, eine zu große Tiefe erzeugt jedoch unter Umständen sehr viel schwerwiegendere Nebenwirkungen. Da im Herzen Gefäßwände verlaufen, die nicht unnötig geschädigt werden dürfen und auch das zu abladierende Gewebe häufig nur eine begrenzte Dicke aufweist, können bei zu großen Tiefen der Läsionen sogar letale Unfälle aufgrund durchtrennter Herzwände oder - gefäße auftreten. Bei herkömmlichen Ablationsverfahren wurde folglich versucht, durch die synchrone Aufzeichnung von EKG-Signalen am eintretenden Behandlungserfolg die optimale Läsionstiefe zu ermessen. Hierbei war jedoch die eingestrahlte Hochfrequenzenergie der Aufzeichnung dieser Signale äußerst abträglich und es wurde der Versuch unternommen, derartige Einflüsse durch entsprechende elektrische oder elektronische Filter in den nachgeschalteten Geräten zu mildern. Diese Versuche waren jedoch nur begrenzt oder nicht erfolgreich. Die Verminderung der eingestrahlten Leistung führte zu extrem langen Behandlungszeiten, welche im Bereich mehrerer Stunden liegen und hierbei sowohl den Patienten erheblich belasten als auch ein Verrutschen des Ablationskatheters nicht sicher vermeiden können. Ferner ist ab einer bestimmten Leistung keine Läsion mehr möglich, da die erzeugte Temperatur für eine Gewebekoagulation nicht mehr ausreicht.
Folglich liegt der Erfindung die Aufgabe zu Grunde, die Aufzeichnung von EKG-Signalen bei der Katheterablation zu ermöglichen und insbesondere die Qualität der aufgezeichneten EKG-Signale soweit zu verbessern, daß medizinische Aussagen in Bezug auf die Herztätigkeit ermöglicht werden.
Diese Aufgabe wurde durch die Erfindung in höchst überraschender Weise mit einem Verfahren zur Herstellung eines Katheters mit verbesserten Eigenschaften nach einem der Ansprüche 1 bis 16 gelöst.
Der Erfinder ging in überraschender Weise einen vollständig anderen Weg als dies beim bekannten Stand der Technik bisher getan wurde.
Es wurden nicht die Geräte zur Aufzeichnung verändert bzw. versucht zu verbessern, sondern es wurde die Ursache der Störungen der Aufzeichnung der EKG-Signale vermindert oder sogar vollständig beseitigt.

Der Erfinder fand erstmalig heraus, daß die Ursache der elektrischen Störungen der EKG-Aufzeichnung bei simultaner Einstrahlung von Hochfrequenzenergie im wesentlichen nicht in den Leitungen zu und von den Katheterelektroden, nicht in den elektronischen Aufzeichnungseinrichtungen und insbesondere nicht in deren Eingangsfiltern liegen sondern in elektrische Störzentren im Bereich der Oberfläche der Ablations- oder Mappingelektroden liegt.

Diese Erkenntnis war um so überraschender als jeglicher untersuchter Ablationskatheter mit Platinelektroden derartige elektrische Störzentren aufwies und nach deren Verminderung oder Entfernung, im wesentlichen nach deren Entfernung von der Elektrodenoberfläche, nahezu oder vollständig frei von den vorstehend beschriebenen unerwünschten Störungen war.

Bei einem Katheter zur Ablation von biologischem, insbesondere von tierischem oder menschlichem Gewebe, vorzugsweise zur Ablation von myokardialem Gewebe des Menschen, mit mindestens einer Ablations- oder Mappingelektrode weist diese mindestens eine Ablations- oder Mappingelektrode eine reduzierte Anzahl elektrischer Störzentren auf. Hierdurch werden beispielsweise die in Fig. 5 und 6 dargestellten, gestörten EKG-Aufzeichnungen derart verbessert, daß die in den Fig.7 bzw. 9 dargestellten Signale erhalten werden können. Überraschend war auch festzustellen, daß bereits die EKG-Signale ohne angelegte Hochfrequenz erheblich verbessert wurden, d.h. deutlich weniger Störsignale aufwiesen.

In besonders vorteilhafter Weise sind die elektrischen Störzentren, welche bei Abgabe von Hochfrequenzenergie an der zumindest einen Ablations- oder Mappingelektrode elektrische Signale erzeugen und die im wesentlichen an Oberflächenbereichen der zumindest einen Ablations- oder Mappingelektrode angeordnet sind in deren Anzahl, flächigen Erstreckung und/oder elektrischen Wirkung vermindert. Hierdurch kommt es zu einer Entfernung oder elektrischen Deaktivierung des Einfluß dieser Störzentren.

Ein besonders wirkungsvolles Verfahren, um die vorstehenden Erfolge zu erzielen besteht darin, daß die zumindest eine Ablations- oder Mappingelektrode eine elektrolytisch behandelte Oberfläche aufweist. Ein elektrolytisches Verfahren zur Verrundung kleiner röhrenförmiger medizinischer Artikel ist aus DE 196 28 879 A1 bekannt. Bei diesem Verfahren wird in den Hohlkörper ein kathodaler Stift mit definiertem Durchmesser eingeführt und durch Anlage eines elektrischen Stromes zwischen Innenstift und einem anodal geschalteten ein elektrisches Potential erzeugt, wobei durch Zugabe eines elektrisch leitendem Elektroyten eine elektrolytische Innenentgratung an Ecken und Kanten erzielt wird.

Bei der elektrolytischen Behandlung, d.h. einer Behandlung mit einem Elektrolyten und angelegter Spannung oder eingeprägtem Strom ist es besonderes vorteilhaft, wenn die Behandlung mit einer Halogenionen, insbesondere Chlorionen, enthaltenden Lösung durchgeführt wird, denn dann sind atomare Umlagerungsprozesse an der Metallinsbesondere an der Platinoberfläche beobachtbar, welche zu einer geänderten Oberflächenstruktur führen, welche die erwünschten positive Eigenschaften aufweist.

Nach dieser Behandlung war häufig zu beobachten, daß Strukturen der Oberfläche der mindestens eine Ablations- oder Mappingelektrode, eine verrundete Oberflächenstruktur aufweisen, deren Kanten einen Radius von mehr als ungefähr 500 nm, vorzugsweise von mehr als 100 nm aber wenigstens mehr als 10 nm aufweisen und es wird vermutet, daß diese Oberflächenveränderungen bereits zumindest einen Teil der Verminderung der elektrischen Störzentren oder deren Wirkungen hervorrufen.

Nach der Behandlung war festzustellen, beispielsweise anhand von optischen Untersuchungen der Verfärbungen einer Platin-Ablationselektrodenoberfläche, daß wenn die zumindest eine Ablations- oder Mappingelektrode ein Metall, umfaßt, dessen Atome an der Oberfläche zumindest bereichsweise atomar oder amorph und im wesentlichen nicht kristallin gebunden vorliegen. Durch diese Umlagerung oder elektrolytische Anlagerung durch galvanische Abscheidungsprozesse wird angenommen, daß an der Oberfläche vorhandene elektrische Potentiale, beispielsweise durch Korngrenzen des kristallin vorliegenden Metalls kompensiert werden und nach der erfindungsgemäßen Behandlung auch mikroskopische elektrische kristalline Potentialdifferenzen, Bereiche mit Feldstärkemaxima oder mikroskopisch unterschiedliche Reaktiosvermögen an der Elektrodenoberfläche ausgeglichen sind. Hierdurch werden die beispielsweise bei der HF-Energieabgabe auftretenden Phänomene, die ohne Beschränkung der Allgemeinheit oder der Breite der Erfindung, lokal verschiedener ionischer Beweglichkeit zugeschrieben werden, gemildert, denn es findet kein "Nachlaufen" stärker gebundener bzw. weniger beweglicher polarer Ionen mehr statt, durch welches elektrische Potentiale gebildet werden, die sich dem EKG-Signal überlagern. Die sich nun an allen Orten der Oberfläche der Ablations- oder Mappingelektrode nahezu gleich bewegenden Ionen erzeugen keine lokalen Feldstärkedifferenzen mehr und stören auch nicht mehr die EKG-Aufzeichnung.

Folglich wird davon ausgegangen, daß wenn der Katheter in vorteilhafter Weise eine Platin-Ablations- oder Mappingelektrode umfasst, die Oberfläche eine Ablations- oder Mappingelektrode zumindest bereichsweise mit elementarem Platin belegt ist. Es liegt im Rahmen der Erfindung, eine derartige atomare, im wesentlichen nichtkristalline oder amorphe Belegung aber auch beispielsweise mit galvanischen Abscheidungs- oder allgemein bekannten Auftragungs- oder Plattierungstechniken zu erzeugen.

In vorteilhafter Weise ergibt es sich dann, daß die Oberfläche der zumindest einen Ablations- oder Mappingelektrode Bereiche mit abgeschiedenem, im wesentlichen amorph oder atomar vorliegendem Metall umfasst.

Bei dem Verfahren zur Herstellung eines Katheters mit verbesserten elektrischen Eigenschaften, bei welchem der Katheter zumindest eine Ablations- oder Mappingelektrode umfasst wird die zu behandelnde Ablations- oder Mappingelektrode des Katheters in eine Lösung eingetaucht, welche Ionen enthält, die durch ein elektrisches Feld in deren Bewegung beeinflußbar sind, dies wird vorteilhaft dadurch erreicht, daß zwischen der zu behandelnden Ablations- oder Mappingelektrode des Katheters und einer weiteren, mit der Lösung in Kontakt stehenden Elektrode eine elektrische Spannung angelegt wird, welche die Bewegung der Ionen erzeugt. Die sich auf die Katheterelektrodenoberfläche zu bewegenden Ionen treffen dort auf und erzeugen sowohl mit deren elektrischen Feldern, wie beispielsweise deren Dipolmoment oder energetischen Potentialen der atomaren oder molekularen Elektronenhülle und deren kinetischer Energie Wechselwirkungen an der Metalloberfläche, welche die erwünschten elektrischen Folgen der atomaren Um- oder Anlagerung meßbar nach sich ziehen.

Besonders vorteilhaft ist das Verfahren durchführbar, wenn die Lösung NaCl in einem Bereich von 0.1 bis 100 g/l enthält. Ferner liegt ein besonders bevorzugter Bereich vor, wenn die Lösung NaCl in einer Menge von etwa 7 g/l enthält.

Anlagerungen an der Ablations- oder Mappingelektrodenoberfläche werden beispielsweise dann erreicht, wenn die Lösung Ionen eines Metallsalzes enthält. Bisherige Oberflächenbearbeitungen, beispielsweise bei Platin-Iridium-Kathetern zielten darauf ab, die Oberfläche zu vergrößern, d.h. gerade nicht zu glätten sondern rauhe Strukturen mit einer um etwa den Faktor 1000 größerer Oberfläche zu schaffen; die Erfindung geht jedoch überraschend erfolgreich gerade den entgegengesetzten Weg.

Gute Ergebnisse werden mit einer angelegten Wechselspannung erreicht, die Anteile enthält, welche eine Frequenz von mehr als 0,01 Hz und weniger als 10 kHz aufweisen. Der besonders bevorzugte Frequenzbereich erstreckt sich von 1 bis 100 Hz und liegt am stärksten bevorzugt etwa bei 10 Hz.

Gute Ergebnisse werden erzielt, wenn die angelegte Wechselspannung in einem Bereich von 0,1 bis 100 Veff liegt. Der am stärksten bevorzugte Bereich ergibt sich, wenn die angelegte Wechselspannung bei 3 bis 7 Ve_{ff} liegt.

An Stelle einer angelegten Spannung kann an der Ablations oder Mappingelektrode und der weiteren Elektrode auch ein Wechselstrom aufgeprägt werden, der eine Spannung mit den in vorstehend aufgeführten Eigenschaften erzeugt. Hierbei ergeben sich die besten Ergebnisse, wenn der Wechselstrom pro Ablations- oder Mappingelektrode eine Stromstärke von etwa 1 mAeff bis 1 Aeff, vorzugsweise von 30 bis 100 mAeff aufweist.

Eine vorteilhafte Vorrichtung zur Katheterbehandlung umfaßt ein Gefäß zur Aufnahme von elektrolytischer Lösung und von Bereichen des Katheters sowie während der Durchführung der Katheterbehandlung eine elektrolytische Lösung, eine Anschlußeinrichtung zum Verbinden mindestens einer Ablations oder Mapping-elektrode des Katheters und einer weiteren Elektrode mit einer spannungs- oder stromerzeugenden Einrichtung, bei welcher die Ablations- oder Mapping-elektrode und die weitere Elektrode während der Durchführung der Behandlung mit dem Elektrolyten benetzbar sind.

Bei einer kompakten, transportablen und vor Ort, direkt vor der Behandlung einsetzbaren Ausführungsform ist die spannungs- oder stromerzeugende Einrichtung eine mit dem Gefäß mechanisch verbundene interne Einrichtung.

Bei einer kostengünstigen stationären Vorrichtung ist die spannungs- oder stromerzeugende Einrichtung eine mit dem Gefäß nicht mechanisch verbundene externe Einrichtung, beispielsweise in externer Labor-Spannungsgenerator.

Die Erfindung wird nachfolgend anhand bevorzugter Ausführungsformen und unter Bezugnahme auf die beigefügten Zeichnungen detaillierter erläutert.
Es zeigen:

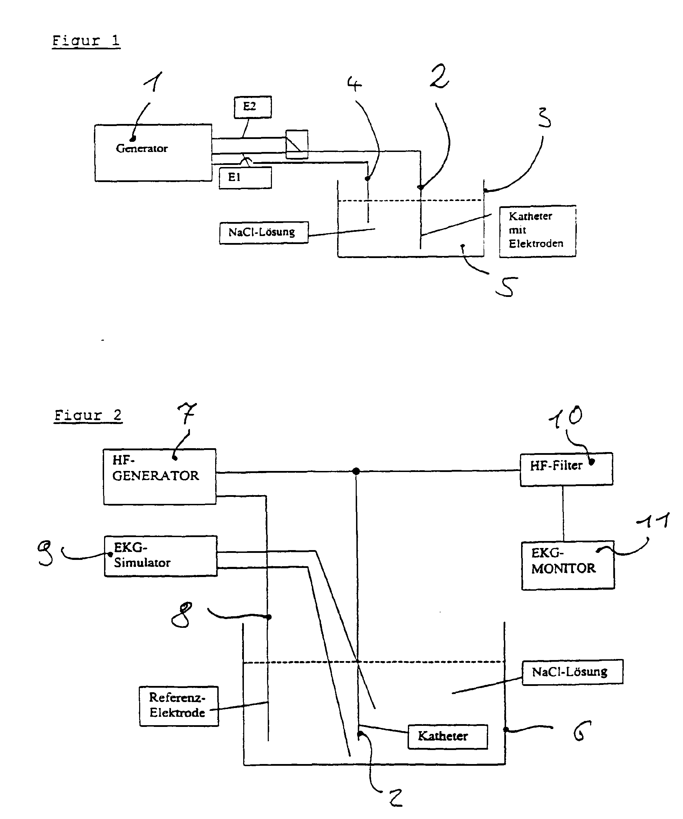

Figur 1 eine schematische Darstellung einer Vorrichtung zur Behandlung von Ablationskathetern,
Figur 2 eine schematische Darstellung einer Vorrichtung zur Messung von simulierten EKG-Signalen mit und ohne eingestrahlte Hochfrequenzenergie,

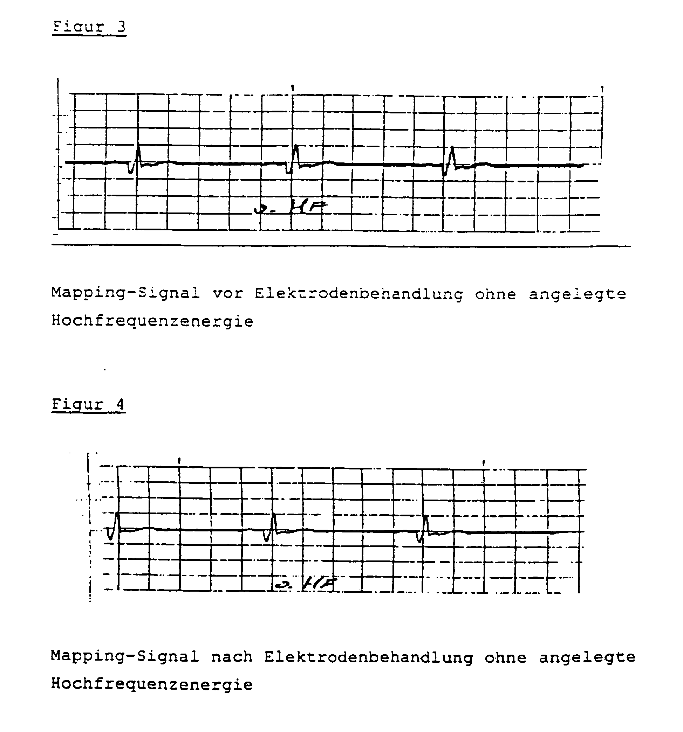

Figur 3 ein simuliertes EKG-Signal, als Mapping-Signal, vor Elektrodenbehandlung ohne angelegte Hochfrequenzenergie,
Figur 4 ein simuliertes EKG Signal, als Mapping-Signal, nach Elektrodenbehandlung ohne angelegte Hochfrequenzenergie,

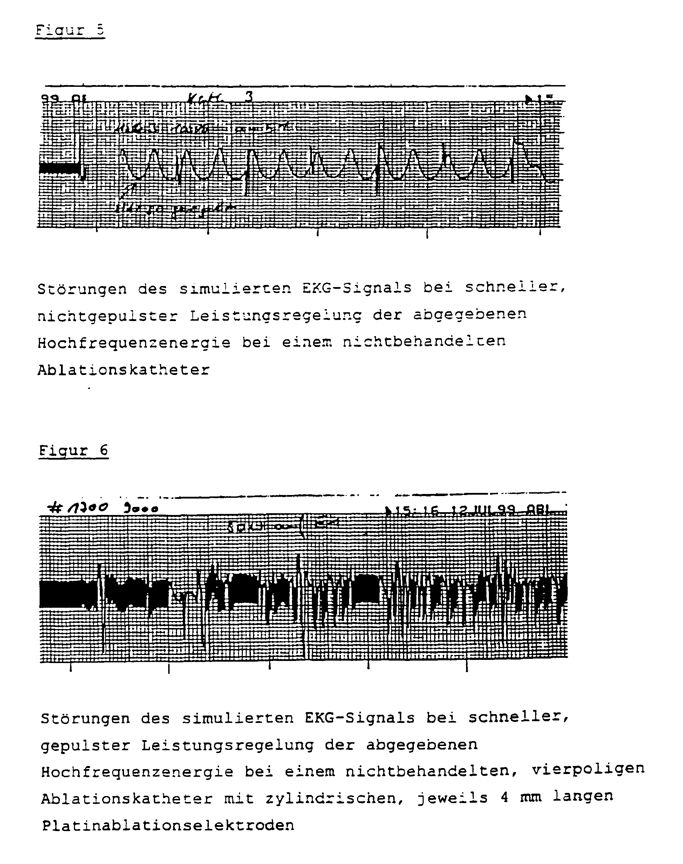

Figur 5 Störungen des simulierten EKG-Signals bei schneller, nichtgepulster Leistungsregelung der abgegebenen Hochfrequenzenergie bei einem nichtbehandelten Ablationskatheter,
Figur 6 Störungen des simulierten EKG-Signals bei schneller, gepulster Leistungsregelung der abgegebenen Hochfrequenzenergie bei einem nichtbehandelten, vierpoligen Ablationskatheter mit zylindrischen, jeweils 4 mm langen Platinablationselektroden,

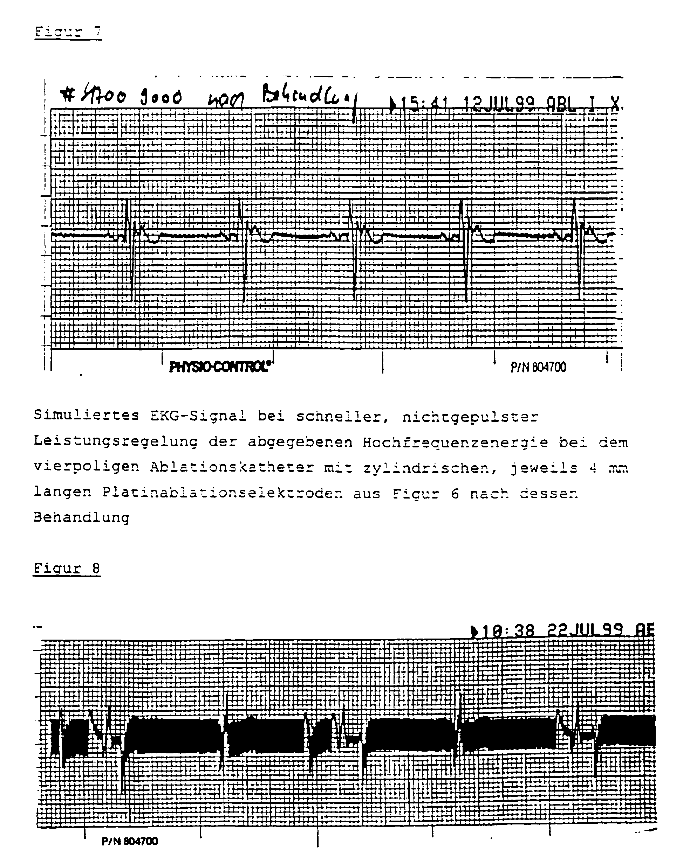

Figur 7 ein simuliertes EKG-Signal bei schneller, nichtgepulster Leistungsregelung der abgegebenen Hochfrequenzenergie bei dem vierpoligen Ablationskatheter mit zylindrischen, jeweils 4 mm langen Platinablationselektroden aus Figur 6 nach dessen Behandlung,
Figur 8 Störungen des simulierten EKG-Signals bei schneller, gepulster Leistungsregelung der abgegebenen Hochfrequenzenergie bei einem nichtbehandelten, Ablationskatheter mit einer zylindrischen, 4 mm langen Platinablationselektrode und drei weiteren Mappingelektroden,

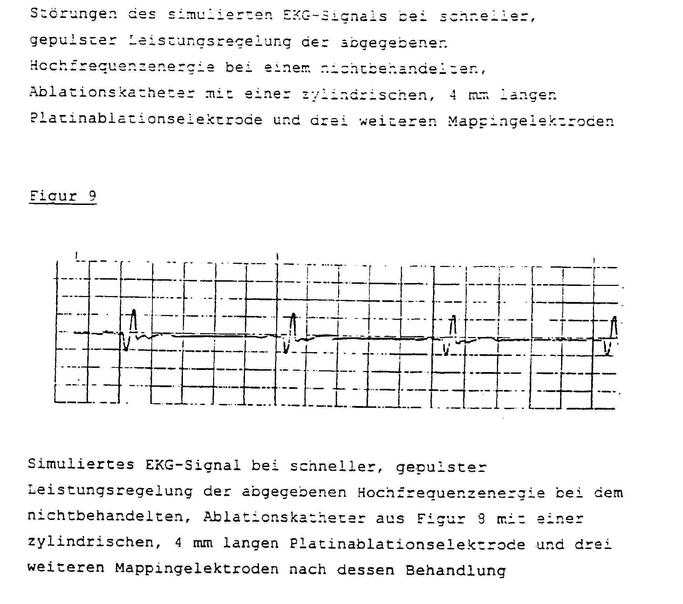

Figur 9 ein simuliertes EKG-Signal bei schneller, gepulster Leistungsregelung der abgegebenen Hochfrequenzenergie bei dem nichtbehandelten, Ablationskatheter aus Figur 8 mit einer zylindrischen, 4 mm langen Platinablationselektrode und drei weiteren Mappingelektroden nach dessen Behandlung,

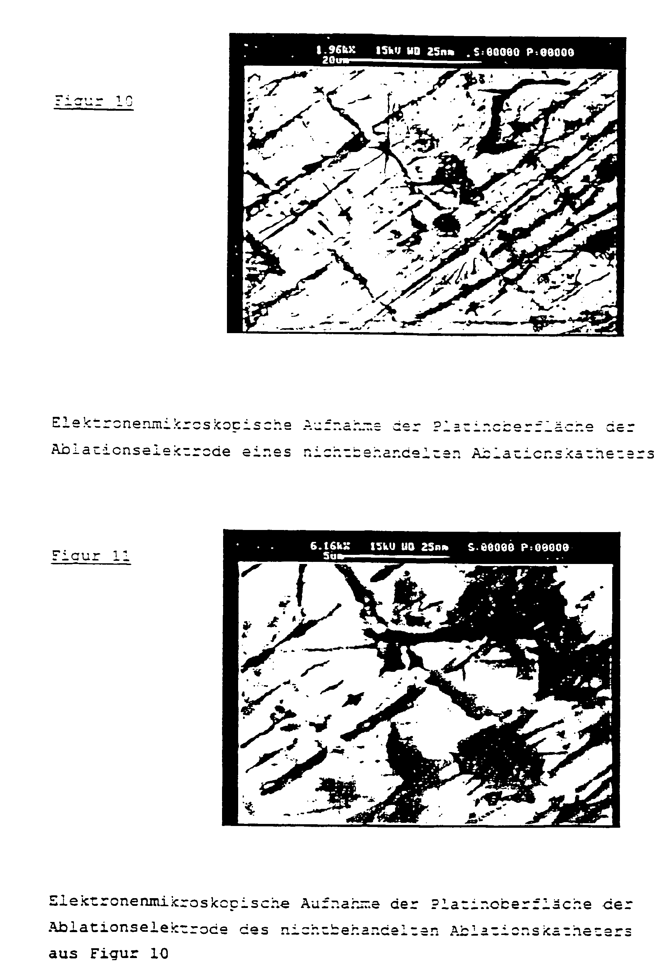

Figur 10 eine elektronenmikroskopische Aufnahme der Platinoberfläche der Ablationselektrode eines nichtbehandelten Ablationskatheters in 1960 facher Vergrößerung

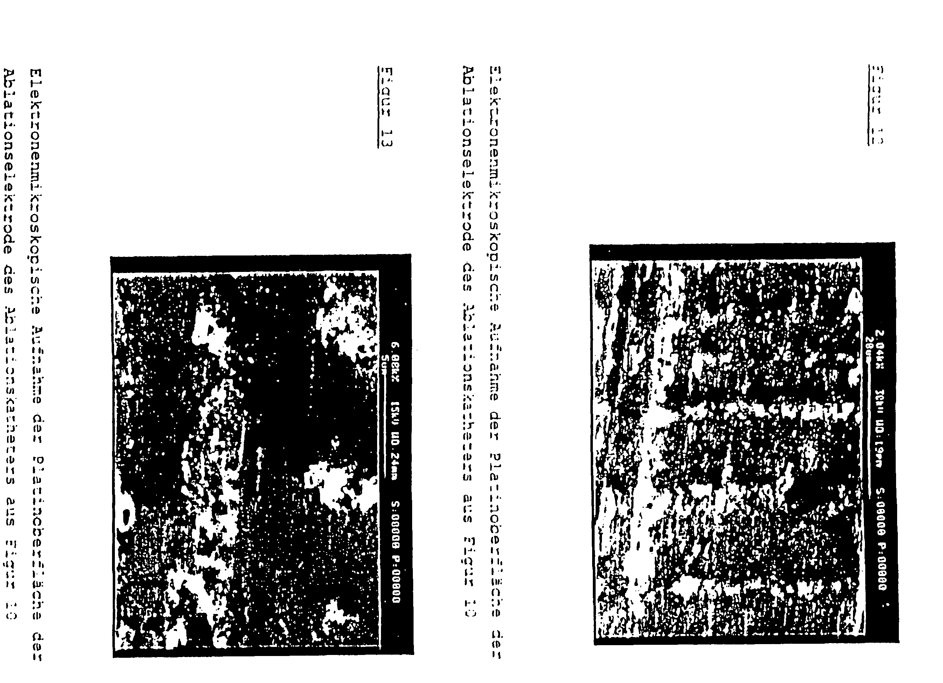

Figur 11 eine elektronenmikroskopische Aufnahme der Platinoberfläche der Ablationselektrode des nichtbehandelten Ablationskatheters aus Figur 10 in 6160 facher Vergrößerung,

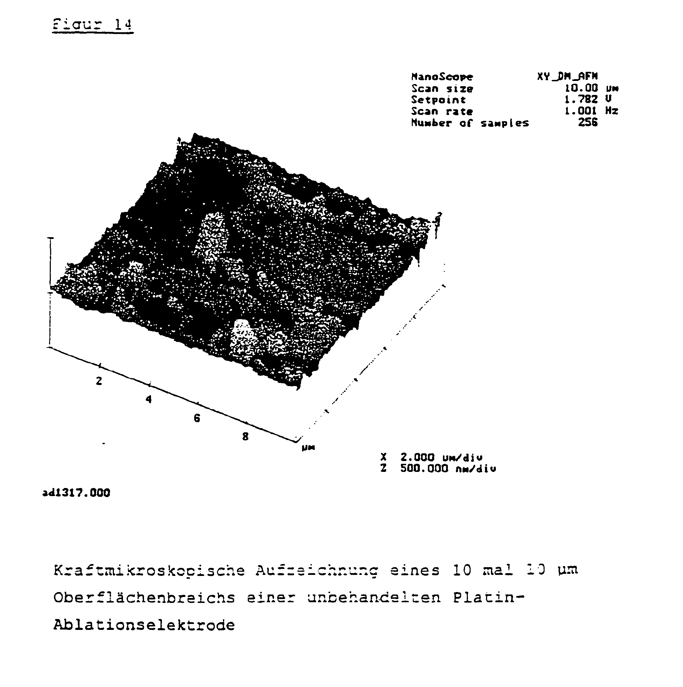

Figur 12 eine elektronenmikroskopische Aufnahme der Platinoberfläche der Ablationselektrode des Ablationskatheters aus Figur 10 in 2040 facher Vergrößerung nach dessen Behandlung,
Figur 13 eine elektronenmikroskopische Aufnahme der Platinoberfläche der Ablationselektrode des Ablationskatheters aus Figur 10 in 6080 facher Vergrößerung nach dessen Behandlung,

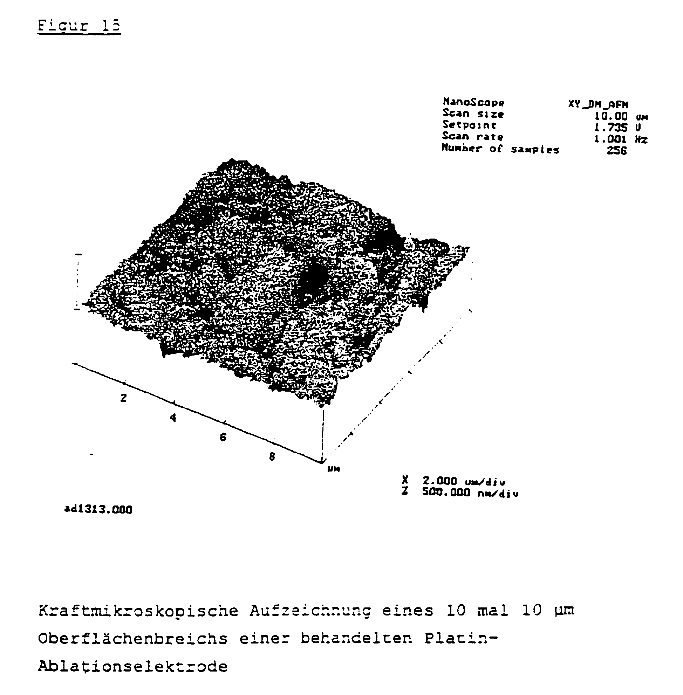

Figur 14 eine'AFM- (atomic force microscopic) bzw. kraftmikroskopische Aufzeichnung eines 10 mal 10 um großen Oberflächenbreichs einer unbehandelten Platin-Ablationselektrode,
Figur 15 eine AFM- bzw. kraftmikroskopische Aufzeichnung eines 10 mal 10 gm großen Oberflächenbreichs einer behandelten Platin-Ablationselektrode.

Die Erfindung wird nachfolgend detaillierter und unter Bezugnahme auf die beigefügten Zeichnungen beschrieben. Zunächst wird auf Fig. 1 Bezug genommen, welcher ein Generator 1, der mit einem Katheter 2 verbunden ist sowie ein elektrolytbefülltes Gefäß 3 zu entnehmen ist.

Der Katheter ist bei dem Beispiel aus Fig. 1 mit zumindest einer Ablations- oder Mappingelektrode, die über eine Zuleitung E1 mit dem Generator 1 verbunden ist, versehen sowie mit einer weiteren Elektrode, welche über eine Zuleitung E2 mit dem Generator 1 verbunden ist. Die weitere Elektrode kann eine Mapping- oder eine Ablationselektrode sein.

Als Katheter eignen sich für die Durchführung der Erfindung im wesentlichen alle bekannten Ablationskatheter, insbesondere Katheter mit Platinelektroden und es wurden bei den Untersuchungen des Erfinders erfolgreichbeispielsweise die nachstehend angegebenen Katheter verwendet:
1. BARD SideWinder Catheter S/N: 17009000
2. BARD SideWinder Catheter S/N: 1300013000
3. Cordis Webster Catheter Intemal S/N: CW1
4. Cardiac Pathways Catheter S/N: G709313
5. Biotronic Catheter: AlCath Twin (nicht Ablations-Katheter, Fraktale Pt/Ir-Oberfläche)
6. BARD Stinger Distal Tip Ablationskatheter 4mm Tip
7. BARD Stinger Distal Tip Ablationskatheter 8mm Tip
8. Biotronic Catheter AlFractal, Distal Tip Ablationskatheter (Fraktale Pt/Ir-Oberfläche)

Als Generator 1 wurde ein herkömmlicher Labor-Wechselstromgenerator verwendet, welcher Frequenzen im Bereich von 0,01 Hz bis 10 kHz erzeugen konnte. Es wurden bei der Behandlung des Katheters 1, welcher bei der vorliegenden Ausführungsform Platinelektroden aufwies, Spannungen in einem Frequenzbereich von 1 bis 100 Hz, vorzugsweise bei 10 Hz liegend angelegt, deren Effektivspannung in einem Bereich von 0,1 bis 100 Veff lagen.

Ein besonders bevorzugter Bereich lag bei 1 bis 10 Veff bzw. der am meisten bevorzugte Wechselspannungsbereich lag bei 3 bis 7 Veff. Alternativ zu dem Spannungsgenerator konnte ein Stromgenerator verwendet werden, der im Bereich von 1mAeff bis 1Ae_{ff}, vorzugsweise in einem Bereich von 30 bis 100mAeff geregelt wurde, wobei diese Stromstärke pro Ablations- oder Mappingelektrode aufgebracht wurde.

Diese Spannung bzw. dieser Strom wurde erzeugt zwischen der zumindest einen Ablations- oder Mappingelektrode des Katheters 2 und der weiteren, über die Zuleitung E2 angeschlossenen Elektrode oder wurde zwischen der über die Zuleitung E1 angeschlossenen Elektrode und einer weiteren, mit der Elektrolytlösung 5 im Kontakt stehenden weiteren Elektrode 4 erzeugt, wobei der Katheter 2 mit den zu behandelnden Elektroden in die Elektrolytlösung 5 eingetaucht war.
Diese Spannungen bzw. Stromstärken wurden über einen Zeitraum von ca. 1 Sekunde bis mehreren Minuten angelegt, wobei durch Messungen in dem in Fig. 2 dargestellten Aufbau gezeigt werden konnte, daß jeweils eine Sättigung erreicht werden konnte, welche mit dem nahezu vollständigen Verschwinden von Störsignalen einherging. Danach brachte eine weitere Behandlung keine merklichen Vorteile mehr.

Ferner war es möglich, auch mehr als eine Ablations- oder Mappingelektrode gleichzeitig zu behandeln, beispielsweise im Falle eines vier Ablationselektroden umfassenden Katheters, bei welchem lediglich die nötige Stromstärke anstieg, um im gleichen Zeitraum bei mehreren Elektroden den gleichen positiven Effekt zu erzeugen. Hierbei konnte sowohl gegen benachbarte Katheterelektroden als auch gegen die weitere Elektrode 4 Spannungen angelegt oder Ströme eingeprägt. werden.

Als Elektrolytlösung wurde eine Halogenionen-enthaltende Lösung verwendet, welche vorzugsweise Chlorionen und in am meisten bevorzugter Weise eine NaCl-Lösung enthielt.

Die Konzentration der NaCl-Lösung lag in einem Bereich von 0,1 bis 100 Gramm pro Liter und lag in bevorzugter Weise bei etwa 7 Gramm pro Liter, welches in etwa einer physiologischen Kochsalzlösung entspricht. Bei niedrigerer Konzentration ergaben sich bei etwa gleich guten Ergebnissen lediglich längere Behandlungszeiten.

Die Katheter wurden im wesentlichen so lange in der Elektrolytlösung 5 belassen, bis sich bei angelegter Wechselspannung der gewünschte störungsverminderte Wert der Signalübertragungsqualität bezogen auf das EKG-Signal bei angelegter Hochfrequenz ergab.

Zur Überprüfung des Ergebnis wurde der in Fig. 2 dargestellte Aufbau verwendet, welcher ein Gefäß 6mit physiologischer NaCl-Lösung enthielt, in welchem der Katheter 2 derart angeordnet war, daß dessen Ablations- oder Mappingelektrode vollständig von der NaCl-Lösung benetzt war, ferner war der Katheter 2 an einen herkömmlichen Hochfrequenzgenerator 7 angeschlossen, mit welchem die bei der Ablation typischen Hochfrequenzenergiewerte der Ablationselektrode des Katheters 2 zugeführt wurden.

Das HF-Feld wurde von dem HF-Generator 7 zwischen der Ablationselektrode des Katheters 2 und einer Referenzelektrode 8 erzeugt, und stellte auf diese Weise in sehr guter Nährung eine Situation dar, wie sie auch beispielsweise im menschlichen Herzen vorliegt.

Mit einem EKG-Simulator 9 wurden Spannungssignale erzeugt, welche in sehr guter Näherung, den vom menschlichen Herzen abgegebenen elektrischen Spannungen sowohl von der Höhe als auch von deren zeitlichem Verlauf entsprachen.

Der Katheter 2 war ferner an ein Hochfrequenzfilter 10 angeschlossen, welcher die vom HF-Generator 7 eingespeisten Hochfrequenz-Signalanteile ausfilterte. Derartige Filteranordnungen sind dem Fachmann wohl bekannt und können beispielsweise den im QuadraPulse-Gerät der AD-Elektronik verwendeten Eingangsfilter entsprechen.

Das vom Katheter abgenommene, insbesondere von dessen Mapping-Elektrode, oder sogar dessen Ablationselektrode gewonnene EKG-Signal wurde dann einem EKG-Monitor 11 zugeführt, wie dieser beispielsweise von der Firma Physiocontrol unter der Bezeichnung LIFEPAK 10 oder von der Firma Bard als EP-Laborsystem vertrieben wird.

Die erhaltenen Ergebnisse werden nachfolgend unter Bezug auf die Figuren 3 bis 9 detaillierter erläutert.

Solange den Katheterelektroden keine Hochfrequenz-Energie bzw. Hochfrequenzspannung zugeführt wurde, weisen die Figuren 3 und 4 nach, daß die Aufzeichnung der EKG-Signale nahezu ungestört vorgenommen werden konnten.

Regelt man jedoch während der EKG-Aufzeichnung die Höhe der Hochfrequenzspannungbzw. die Menge an eingestrahlter Hochfrequenz-Energie wie es während eine realen Ablationsvorgangs am Patienten der Fall ist, so kommt es zu Spannungen, welche nahezu linear proportional zur eingestrahlten Energie verlaufen und beispielsweise in Fig. 5 dargestellt sind.

Eine Regelung der abgegebenen Energie im Wege einer. Leistungsregelung der eingestrahlten Hochfrequenz-Energie führt somit stets zu einer Störsignalüberlagerung der EKG-Signale, welche es dem Mediziner in der Regel unmöglich machen, eine Aussage. über den Behandlungserfolg oder den aktuellen Zustand des Herzens zu treffen.

Noch schwieriger ist die Situation bei gepulster Leistungsregelung, wie dies in den Fig. 6 und 8 dargestellt ist, in welchen nahezu überhaupt keine Anteile des EKG-Signals mehr zu erkennen sind.

Die bei diesen Versuchen eingestrahlte Hochfrequenzleistung lag bei etwa 1 bis 50 W, wie es bei der Hochfrequenz-Katheterablationim menschlichen Herzen durchaus üblich ist.

Wurde ein Abblationskatheter jedoch auf die vorstehend beschriebene Weise behandelt, so konnten die überlagerten Störungen bei gleichem Versuchsaufbau bis auf einen nahezu nicht mehr meßbaren Wert, jedenfalls um eine Faktor von mehr als zehn reduziert werden, wie es beispielsweise in den Figuren 7 und 9 dargestellt ist.

Die in Fig. 7 dargestellte EKG-Ableitung entspricht im wesentlichen dem Aufbau und den jeweiligen Werten, die bei einem unbehandelten Katheter zu den in Fig. 5 gezeigten Ergebnissen führt, während die in Fig. 9 dargestellten Ergebnisse, welche bei einem behandelten Katheter gewonnen wurden, denjenigen entsprachen, die in den Figuren .6 und 8 für den unbehandelten Katheter gezeigt wurden.
Der an sich jeweils identische Versuchsaufbau, der sich lediglich darin unterschied, ob der Katheter direkt so, wie er vom jeweiligen Hersteller vertrieben wurde, benutzt wurde oder ob dieser in erfindungsgemäßer Weise behandelt wurde, weist den großen Erfolg der vorliegenden Erfindung unzweideutig nach.
Die Katheter verfügen folglich an deren Elektrodenoberflächen über weniger elektrische bzw. elektronische Störzentren, welche die überlagerten Signale erzeugen können. Das Maß der Störungsverminderung ist folglich ein Maß für das Vorhandensein bzw. verminderte oder abgeschwächte Vorhandensein derartiger Störzentren. Zur Erzeugung derartiger, dem EKG-Signal überlagerter, Signale wird ohne Beschränkung der Allgemeinheit und ohne Beschränkung der Erfindung angenommen, daß es zu lokalen Haftstellen bzw. lokalen Extrema der elektrischen Feldstärke an der Oberfläche des Katheters kommt, an welcher Ionen bzw. Moleküle mit Dipolmoment unterschiedlich stark gebunden bzw. beschleunigt werden können, die dann beim Anlegen der HF-Spannung bzw. -energie durch die verschiedene Beweglichkeit ein Spannungssignal erzeugen können, welches sich dem EKG-Signal überlagert.
Zum Nachweis eines derartigen Verhaltens wurden die in den Figuren 10 bis 13. dargestellten elektronenmikroskopischen Aufnahmen gewonnen, welche zeigen, wie beispielsweise Figuren 10 und 11, daß die zunächst im Mikrostrukturbereich scharfkantige Katheteroberfläche nach der elektrolytischen Behandlung weiche Verrundungen und weniger scharfe Riefen oder Rillen aufweist.

Allein durch die mechanische Glättung kann die mechanische Reibung der Ionen an der Oberfläche reduziert werden, so daß hierdurch bedingte mechanisch bewirkte jedoch elektrisch wirksame Störzentren vermindert werden.

Ferner konnte an der behandelten Oberfläche der Ablations- oder Mappingelektrode durch optische Untersuchungen die Abscheidung oder das Vorhandensein elementaren Platins nachgewiesen werden. Dies führte zu der *Annahme,* daß kristalline Korngrenzen oder andere geeignet Oberflächenbereiche des Platins, beispielsweise Bereiche mit spitzen Kanten und hohen elektrischen Felsdstärken vom Angriff der Chlorionen betroffen werden und Platin- bzw. Metallatome herausgelöst werden können. Durch die kinetische Energie und/oder die Potentiale der Elektronenhülle der Chlorionen kann es zum Ablösen von Platinatomen aus dem metallischen kristallinen Verbund und deren amorpher Umlagerung kommen.

Auch eine virtuelle Ablösung, d.h. eine Migration im gebundenen Zustand des Platinatoms führt zu einer Loslösung des Atoms aus dem Kristallverbund und dessen Umlagerung.

Hierdurch lassen sich auch die einem erhöhten Angriff ausgesetzten verrundeten Spitzen der behandelten Oberfläche erklären, denn gerade in diesen Bereichen kann ein Angriff von mehreren Seiten her erfolgen.

Eine weitere alternative Erklärung besteht darin, daß es durch die Halogenionen zu dem aus der Vakuumbearbeitung von Halbleitern bekannten Ion-Milling, "Ionenmahlen", kommt, bei welchem ein mechanischer Abtrag an der Oberfläche stattfindet.

Ganz besonders deutlich wird der durch die Behandlung hervorgerufene Unterschied auch aus kraftmikroskopischen Aufzeichnungen, welche beispielsweise in Fig. 14 die unbehandelte. Oberfläche mit zapfenartigen Fortsätzen und scharfen Riefen zeigt und im Falle der behandelten Oberfläche, welche in Fig.. 15 dargestellt ist, eine stark geglättete Oberfläche ohne zapfenartige Fortsätze zeigt.

Durch diese Migration von Platinatomen können auch an der Oberfläche befindliche Potentiale, etwa an Korngrenzen, oder Feldstärkemaxima kompensiert werden, so daß auch die effektive elektrische Wirkung derartiger Festkörperpotentiale oder Feldstärkemaxima drastisch reduziert werden kann.

Somit sind die vor der Behandlung vorliegenden elektrischen Störzentren nicht nur in deren flächiger Erstreckung sondern auch in deren elektrischer Wirkung vermindert.

Die Erfinder fanden auch heraus, daß in vielen Fällen bei einem behandelten Katheter Strukturen der Oberfläche der Ablations- oder Mappingelektrode keine scharfen Kanten, d.h. keine sehr kleine Krümmungsradien mehr aufweisen. In einem Oberflächenabschnitt mit einer Länge, Breite oder Höhe von weniger als 10 µm hatten die vorhandenen Kanten einen Radius von mehr als ungefähr 10 bis 50 µm.

Schärfere Kanten bzw. kleinere Radien sind in deren Anzahl regelmäßig entweder vermindert oder treten gar nicht mehr auf. Gemäß der Erfindung betragen die meisten Krümmungsradien der Kanten mehr als ungefähr 500 nm, vorzugsweise mehr als 100 nm aber wenigstens mehr als 10 nm.

Es liegt ferner im Rahmen der Erfindung, anstelle der Halogenionen oder zusätzlich zu der Halogenionenenthaltenden Elektrolytlösung Metallsalze zu lösen, um auf diese Weise zu einer galvanischen amorphen Abscheidung von Metallatomen an der metallischen Ablations- oder Mappingelektrode zu gelangen.

Es sei darauf hingewiesen, daß erfindungsgemäß behandelte Katheter auch bei nicht angelegter Hochfrequenzenergie eine deutlich verbesserte Signalqualität, d.h. wesentlich geringere Störsignale aufweisen. Diese Verbesserung beschränkt sich nicht auf Ablationselektroden sondern kann auch bei Mapping-Elektroden oder -Kathtetern erfolgreich genutzt werden.

## Patentansprüche

1. Verfahren zur Herstellung eines Katheters mit verbesserten elektrischen Eigenschaften, bei welchem der Katheter zumindest eine Ablations- oder Mappingelektrode umfaßt,
**dadurch gekennzeichnet, daß**
die zu behandelnde Ablations- oder Mappingelektrode des Katheters in eine Lösung eingetaucht wird, welche Ionen enthält, die durch ein elektrisches Feld in deren Bewegung beeinflußbar sind,
zwischen der zu behandelnden Ablations- oder Mappingelektrode des Katheters und einer weiteren, mit der Lösung in Kontakt stehenden Elektrode eine elektrische Spannung angelegt wird.

2. Verfahren nach Anspruch 1,
**dadurch gekennzeichnet, daß** die weitere Elektrode eine Elektrode des Katheters ist.

3. Verfahren nach Anspruch 1,
**dadurch gekennzeichnet, daß** die weitere Elektrode eine externe Elektrode ist.

4. Verfahren nach einem der vorstehenden Ansprüche,
**dadurch gekennzeichnet, daß** die Lösung Halogenionen enthält.

5. Verfahren nach Anspruch 4,
**dadurch gekennzeichnet, daß** die Lösung Chlorionen enthält.

6. Verfahren nach einem der vorstehenden Ansprüche,
**dadurch gekennzeichnet, daß** die Lösung NaCl in einem Bereich von 0.1 bis 100 g/l enthält.

7. Verfahren nach Anspruch 6,
**dadurch gekennzeichnet, daß** die Lösung NaCl in einer Menge von etwa 7 g/l enthält.

8. Verfahren nach einem der vorstehenden Ansprüche,
**dadurch gekennzeichnet, daß** die Lösung Ionen eines Metallsalzes enthält.

9. Verfahren nach einem der vorstehenden Ansprüche,
**dadurch gekennzeichnet, daß** die angelegte Spannung eine Wechselspannung ist.

10. Verfahren nach Anspruch 9,
**dadurch gekennzeichnet, daß** die angelegte Wechselspannung Anteile enthält, die eine Frequenz von mehr als 0,01 Hz und weniger als 10 kHz aufweisen.

11. Verfahren nach Anspruch 9 oder 10,
**dadurch gekennzeichnet, daß** die angelegte Wechselspannung Anteile enthält, die in einem Frequenzbereich von 1 bis 100 Hz, vorzugsweise bei 10 Hz liegen.

12. Verfahren nach einem der vorstehenden Ansprüche,
**dadurch gekennzeichnet, daß** die angelegte Wechselspannung in einem Bereich von 0,1 bis 100 V_{eff} liegt.

13. Verfahren nach Anspruch 20,
**dadurch gekennzeichnet, daß** die angelegte Wechselspannung in einem Bereich von 1 bis 10 V_{eff} liegt.

14. Verfahren nach Anspruch 11,
**dadurch gekennzeichnet, daß** die angelegte Wechselspannung bei 3 bis 7 V_{eff} liegt.

15. Verfahren nach einem der Ansprüche von 1 bis 8,
**dadurch gekennzeichnet, daß** an der Ablations- oder Mappingelektrode und der weiteren Elektrode ein Wechselstrom aufgeprägt wird, der eine Spannung mit den in den Ansprüchen 9 bis 14 aufgeführten Eigenschaften erzeugt.

16. Verfahren nach Anspruch 15,
**dadurch gekennzeichnet, daß** der Wechselstrom pro Ablations- oder Mappingelektrode eine Stromstärke von 1 mA_{eff} bis 1 A_{eff}, vorzugsweise von 30 bis 100 mA_{eff} aufweist.

## Claims

1. Method of producing a catheter with improved electrical properties, wherein the catheter comprises at least one ablation or mapping electrode,
**characterised in that**
the ablation or mapping electrode of the catheter to be treated is immersed in a solution which contains ions which can be influenced in their movement by an electrical field,
an electric voltage is supplied between the ablation or mapping electrode of the catheter to be treated and a further electrode in contact with the solution.

2. Method according to claim 1,
**characterised in that** the further electrode is an electrode of the catheter.

3. Method according to claim 1,
**characterised in that** the further electrode is an external electrode.

4. Method according to one of the preceding claims,
**characterised in that** the solution contains halogen ions.

5. Method according to claim 4,
**characterised in that** the solution contains chlorine ions.

6. Method according to one of the preceding claims,
**characterised in that** the solution contains NaCl in a range of 0.1 to 100 g/l.

7. Method according to claim 6,
**characterised in that** the solution contains NaCl in a quantity of around 7 g/l.

8. Method according to one of the preceding claims,
**characterised in that** the solution contains ions of a metal salt.

9. Method according to one of the preceding claims,
**characterised in that** the voltage supplied is an alternating voltage.

10. Method according to claim 9,
**characterised in that** the alternating voltage supplied contains portions which have a frequency of more than 0.01 Hz and less than 10 Hz.

11. Method according to claim 9 or claim 10,
**characterised in that** the alternating voltage supplied contains portions which lie in a frequency range of 1 to 100 Hz, preferably 10 Hz.

12. Method according to one of the preceding claims,
**characterised in that** the alternating voltage supplied lies in a range of 0.1 to 100 V_{eff}.

13. Method according to claim 20,
**characterised in that** the alternating voltage supplied lies in a range of 1 to 10 V_{eff}.

14. Method according to claim 11,
**characterised in that** the alternating voltage supplied represents 3 to 7 V_{eff}.

15. Method according to one of the claims 1 to 8,
**characterised in that** an alternating current is produced on the ablation or mapping electrode and the further electrode which generates a voltage with the properties mentioned in claims 9 to 14.

16. Method according to claim 15,
**characterised in that** the alternating current per ablation or mapping electrode has a current strength of 1 mA_{eff} to 1 A_{eff}, preferably 30 to 100 mA_{eff}.

## Revendications

1. Procédé de fabrication d'un cathéter présentant des propriétés électriques améliorées, dans lequel le cathéter comprend une électrode d'ablation ou de cartographie,
**caractérisé en ce que**
l'électrode d'ablation ou de cartographie à traiter du cathéter est plongée dans une solution qui contient des ions qui peuvent être influencés par un champ électrique dans leur déplacement,
une tension électrique est appliquée entre l'électrode d'ablation ou de cartographie à traiter du cathéter et une autre électrode mise en contact avec la solution.

2. Procédé selon la revendication 1,
**caractérisé en ce que** l'autre électrode est une électrode du cathéter.

3. Procédé selon la revendication 1,
**caractérisé en ce que** l'autre électrode est une électrode externe.

4. Procédé selon l'une quelconque des revendications précédentes,
**caractérisé en ce que** la solution contient des ions halogénés.

5. Procédé selon la revendication 4,
**caractérisé en ce que** la solution contient des ions de chlore.

6. Procédé selon l'une quelconque des revendications précédentes,
**caractérisé en ce que** la solution contient du NaCl dans une plage de 0,1 à 100 g/l.

7. Procédé selon la revendication 6,
**caractérisé en ce que** la solution contient du NaCl en une quantité d'environ 7 g/l.

8. Procédé selon l'une quelconque des revendications précédentes,
**caractérisé en ce que** la solution contient des ions d'un sel de métal.

9. Procédé selon l'une quelconque des revendications précédentes,
**caractérisé en ce que** la tension appliquée est une tension alternative.

10. Procédé selon la revendication 9,
**caractérisé en ce que** la tension alternative appliquée contient des fractions qui présentent une fréquence de plus de 0,01 Hz et moins de 10 kHz.

11. Procédé selon la revendication 9 ou 10,
**caractérisé en ce que** la tension alternative appliquée contient des fractions qui se situent dans une plage de fréquences de 1 à 100 Hz, de préférence aux environs de 10 Hz.

12. Procédé selon l'une quelconque des revendications précédentes,
**caractérisé en ce que** la tension alternative appliquée se situe dans une plage de 0,1 à 100 V_{eff}.

13. Procédé selon la revendication 20,
**caractérisé en ce que** la tension alternative appliquée se situe dans une plage de 1 à 10 V_{eff}.

14. Procédé selon la revendication 11,
**caractérisé en ce que** la tension alternative appliquée se situe aux environs de 3 jusqu'à 7 V_{eff}.

15. Procédé selon l'une quelconque des revendications 1 à 8,
**caractérisé en ce qu'**un courant alternatif, qui génère une tension présentant les propriétés mentionnées dans les revendications 9 à 14, est appliqué à l'électrode d'ablation ou de cartographie et l'autre électrode.

16. Procédé selon la revendication 15,
**caractérisé en ce que** le courant alternatif présente, par électrode d'ablation ou de cartographie, une intensité de courant de 1 mA_{eff} à 1 A_{eff}, de préférence de 30 à 100 mA_{eff}.
